# EUROPÄISCHE PATENTSCHRIFT

(11) **EP 3 295 177 B1**
(45) Veröffentlichungstag und Bekanntmachung des Hinweises auf die Patenterteilung: **26.12.2018**
(21) Anmeldenummer: 16727311.9
(22) Anmeldetag: 08.05.2016
(51) Int. Cl.: G01N 33/68

(54) **VERFAHREN ZUR IDENTIFIZIERUNG VON MARKERPROTEINEN ZUR DIAGNOSE UND RISIKOSTRATIFIZIERUNG VON STÖRUNGEN DER BLUTGERINNUNG**
METHOD FOR IDENTIFYING MARKER PROTEINS FOR DIAGNOSIS AND RISK STRATIFICATION OF BLOOD COAGULATION DISORDERS
PROCÉDÉ D'IDENTIFICATION DE PROTÉINES MARQUEURS DESTINÉES AU DIAGNOSTIC ET STRATIFICATION DE RISQUES DES TROUBLES DE LA COAGULATION SANGUINE

(30) Priorität: 08.05.2015 EP 15166935
(43) Veröffentlichungstag der Anmeldung: 21.03.2018
(73) Patentinhaber: Leibniz - Institut für Analytische Wissenschaften - ISAS - e.V., 44139 Dortmund (DE)
(72) Erfinder: BECK, Florian, 44369 Dortmund (DE); SICKMANN, Albert, 44143 Dortmund (DE); ZAHEDI, René, 44139 Dortmund (DE)
(74) Vertreter: Simandi, Claus
(86) Internationale Anmeldenummer: PCT/EP2016/060245
(87) Internationale Veröffentlichungsnummer: WO 2016/180742

(56) Entgegenhaltungen:
- WO-A1-2011/042467
- WO-A2-01/96594
- WO-A2-2008/131261
- US-A1- 2005 130 230
- US-A1- 2006 172 429
- US-A1- 2010 304 406
- BECK F ET AL: "Time-resolved characterization of cAMP/PKA-dependent signaling reveals that platelet inhibition is a concerted process involving multiple signaling pathways", e-BLOOD, Bd. 123, Nr. 5 30. Januar 2014 (2014-01-30), Seiten E1-E9, XP055224605, DOI: 10.1182/blood-2013-07-512384 Gefunden im Internet: URL:http://www.bloodjournal.org/content/bl oodjournal/123/5/e1.full.pdf [gefunden am 2015-10-29] in der Anmeldung erwähnt
- U. LEWANDROWSKI ET AL: "Platelet membrane proteomics: a novel repository for functional research", BLOOD, Bd. 114, Nr. 1, 2. Juli 2009 (2009-07-02), Seiten e10-e19, XP055224583, US ISSN: 0006-4971, DOI: 10.1182/blood-2009-02-203828

## Beschreibung

Die Erfindung betrifft ein Verfahren zur Identifizierung von Markerproteinen zwecks Diagnose und Risikostratifizierung von Störungen der Blutgerinnung.

Zwecks einer geeigneten Therapie, bedarf es einer frühen Diagnose und Differenzierung von Störungen der Blutgerinnung in Verbindung mit der Notwendigkeit klinische Entscheidungen zu treffen, insbesondere in der Notfallmedizin.

Durch Einwirken verschiedener Substanzen (Kollagen, Thrombin, Immunkomplexe u. a.) sezernieren Thrombozyten in Granula gespeicherte Plättchenfaktoren (Degranulation), die vor allem die Aktivierung des endogenen Systems der Blutgerinnung bzw. Hämostase unter Ausbildung eines Thrombus bewirken. Jedoch kann die Aktivierung der Thrombozyten, insbesondere aus genetischen oder krankhaften Gründen gestört sein. Dies geht zumeist mit einem erhöhten Risiko von schweren kardiovaskulären oder sekundären Effekten einher. Daher ist es erforderlich, den Aktivierungsgrad und Aktivierbarkeit (AuA) von Thrombozyten zu bestimmen. Diese ist mit dem Phosphorylisierungsgrad von Proteinen in Thrombozyten verknüpft, wie z.B. VASP und unterliegt einer Kinaseaktivität.

Die individuelle Bestimmung von AuA von Thrombozyten soll dazu beitragen, eine genauere Klassifizierung von Patienten in Risikogruppen zu ermöglichen, eine geeignete personalisierte Therapie (Präparat, Dosierung, Dauer, Kombination) durchzuführen und diese zudem zu steuern.

Im Stand der Technik werden AuA von Thrombozyten in zahlreichen Thrombozyten-Funktionstests analysiert, wobei Thrombozyten mit Agonisten oder über Scherkräfte aktiviert werden, und z.B. die Aggregation zu bestimmen, um eine Aussage über die Aktivierbarkeit (synonym: engl. Response) von Thrombozyten zu erhalten, wie z.B. nach Behandlung mit Medikamenten, wie Copidogrel.

Diese Funktionstests umfassen vor allem drei Haupttypen: (i) Aggregations-basierte Tests wie z.B. LTA (engl. light transmission aggregometry), (ii) Scherkraft-abhängige Tests und (iii) Durchflusszytometrie. Generell zeigt sich, dass aufgrund der großen Anzahl verschiedener Verfahren und der geringen Standardisierung der Tests (sowie Präanalytik) zum einen die Korrelation zwischen verschiedenen Tests gering ist, und zum anderen die Aussagekraft der einzelnen Tests bezüglich Vorhersage von Medikamenten-Response und Therapiesteuerung in der Praxis unbefriedigend ist (Krisha et al., Circulation 2012, 1288-1303). Auch die Therapiesteuerung mittels Gen-Typisierung wird diskutiert - so dass beide Verfahren derzeit kritisch gesehen werden (Krisha et al., Circulation 2012, 1288-1303). Der VASP Assay (Testsystem zum Nachweis der Aktivität von cyclo-Nulceotid-abhängigen Proteinkinasen, DE 100 29 210 A1), welcher den Phosphorylierungsgrad des VASP Proteins über phosphospezifische Antikörper bestimmt, ist derzeit einer der Goldstandards um aus geringen Blutmengen die Thrombozyten-Funktionsstatus/Aktivierungsstatus zu überprüfen.

Es scheint, dass die Messung eines einzelnen Kinasesubstrats bzw. Parameters, wie in den meisten Tests verwendet, nicht ausreicht um die AuA von Thrombozyten eindeutig zu bestimmen - vor allem im Kontext verschiedener Aktivierungs- sowie Inhibierungssignalwege, die in Thrombozyten bekannt sind.

Nachteilig an den bekannten Diagnoseverfahren ist daher, dass eine frühzeitige und vollständige Erfassung von Risikopatienten nicht ausreichend gelingt und daher eine Risikostratifizierung als auch Therapiesteuerung nicht ausreichend erfolgt. Eine der Erfindung zugrunde liegende Aufgabe besteht daher darin, ein Verfahren zur Risikostratifizierung von Störungen der Blutgerinnung zu entwickeln, das eine verbesserte Erfassung von Risikopatienten ermöglicht als auch in einer weiteren Aufgabe entsprechende Markerproteine zu identifizieren.

Ferner ist nachteilig, dass im Stand der Technik zumeist keine hinreichende Sensitivität und/oder Spezifität der Markerproteine erreicht wird.

Daher ist es Aufgabe der vorliegenden Erfindung ein Verfahren zur Identifizierung von Markerproteinen zwecks Diagnose und Risikostratifizierung von Störungen der Blutgerinnung bereitzustellen als auch ein Verfahren zur Bestimmung der Aktivierungsgrad und Aktivierbarkeit von Thrombozyten, welches aus Blut von einem Patienten (synonym: Proband) durchgeführt wird.

Die Aufgabe wird durch ein Verfahren gemäß Anspruch 1 gelöst, mit den folgenden Schritten:
i.) Bereitstellung von Blut, Vollblut, Blutserum oder Blutplasma enthaltend Thrombozyten,
ii.) Aliquotierung in mindestens zwei Aliquote,
iii.)
   A. Zugabe eines Thrombozyten-Aktivators in mindestens ein erstes Aliquot und
   B. Zugabe eines Thrombozyten-Inhibitors in mindestens ein zweites Aliquot, und ein oder mehrmalige Wiederholung der jeweiligen Zugabe, und optional
   C. Zugabe eines Thrombozyten-Aktivators und Zugabe eines Thrombozyten-Inhibitors in mindestens ein drittes Aliquot oder umgekehrt, und ggfs. ein oder mehrmalige Wiederholung der jeweiligen Zugabe,
iv.) Lyse der Zellen, insbesondere Thrombozyten aus iii.) A. B. und C. im jeweiligen Aliquot,
v.) Extraktion der Proteine und Verdau zu Peptiden mittels Proteasen,
vi.) Anreicherung und Abtrennung der phosphorylierten Peptide,
vii.) Analyse mittels quantitativer Massenspektrometrie und Auswertung,
   wobei solche phosphorylierte Peptide ausgewählt werden, die zugleich eine erhöhte oder erniedrigte Phosphorylierung aus iii.) A in der Zeit zeigen und eine gegenläufige erniedrigte oder erhöhte Phosphorylierung aus iii.) B in der Zeit zeigen und optional im Vergleich gegen phosphorylierten Peptiden aus iii.) C eine erniedrigte oder erhöhte Phosphorylierung zeigen.
(nachstehend: erfindungsgemäßes Verfahren)

Die Schritte iii.) bis vii.) können einzeln oder gemeinsam beliebig oft wiederholt werden. Die Zugaben in iii.) C. können z.B. zeitversetzt in 30 Sekunden erfolgen. Ferner sind die Schritte allesamt oder einzeln automatisierbar.

Zur Diagnose oder Risikostratifizierung ist lediglich erforderlich, dass ein oder mehrere Markerproteine identifiziert werden, die relativ zueinander gemäß iii.) A und iii.) B eine unterschiedliche und zwar entgegengesetzte Phosphorylierung zeigen, und zwar entweder eine jeweils erhöhte *(erniedrigte)* Phosphorylierung in iii.) A (Gegenwart eines Thrombozyten-Aktivator) und erniedrigte *(erhöhte)* Phosphorylierung in iii.) B (Gegenwart eines Thrombozyten-Inhibitor) oder umgekehrt (in Klammern, kursiv). In einer Probe zeigt erfindungsgemäß jeweils ca. 1% der Gesamtproteine dieses Verhalten.

Zu Schritt i.): Bevorzugt ist Blut in Form von Frischblut einer Probe eines Patienten oder Probanden, z.B. im Wege der Blutentnahme (Fingerbeere etc.).

Zu Schritt ii.): Die Aliquotierung (Aufteilung der Probe) kann in beliebig viele Aliquots (Tubes) erfolgen (z.B. 360 oder mehr).

In Schritt iii.) sind geeignete Thrombozyten-Aktivatoren solche wie ADP, Kollagen, Thrombin, Immunkomplexe, Thrombozytenaggregationshemmer u.a. Clopidogrel, Prasugrel oder Acetylsalicylsäure.

In Schritt iii.) sind geeignete Thrombozyten-Inhibitoren solche wie Iloprost (Prostazyklin-Analogon) sowie NO (Stickstoffmonoxid)-Donatoren wie Molsidomin oder Nitroprussid.

Das dritte Aliquot iii.) C. dient zur weiteren Absicherung der Ergebnisse und kann optional hinzugezogen werden.
Des Weiteren kann optional eine Blindprobe D. (Aliquot aus ii.)) zur Kontrolle beibehalten werden, indem keine Zugabe von Thrombozyten-Aktivatoren oder Thrombozyten-Inhibitoren im Schritt iii.) erfolgt, jedoch die Schritte iv.) bis vii.) durchgeführt werden.

In Schritt iv.) kann die Lyse mit beliebigen Methoden erfolgen, insbesondere vorzugsweise mit einem Lysepuffer (2% SDS samt Phosphataseinhibitoren), genaue Zusammensetzung nach Beck et al., Blood 2014, 123(5):e1-e10. Die Lyse bewirkt ein Stoppen der zellulären Signalantwort oder "Einfrieren" der Thrombozytenaktivität.

In Schritt v.) kann die Extraktion mit beliebigen Methoden erfolgen, insbesondere vorzugsweise mit Hilfe einer Ethanol-Präzipitation oder Filter-aided sample preparation (Wisniewski et al., Nat Meth, 6 (2009) 359-362)). Ein proteolytischer Verdau kann z.B. mittels Trypsin erfolgen, (Dickhut et al., J Proteome Res 2014,6;13(6):2761-70), wodurch Peptide mit einer durchschnittlichen Länge von 14 Aminosäuren erhalten werden, die gut massenspektrometrisch detektier- und quantifizierbar sind (bevorzugter Bereich zwischen 7 und 35 Aminosäuren).

Zur Anreicherung der erhaltenen Peptide zwecks quantitativer Massenspektrometrie in Schritt vi.) können die Peptide gelabelt oder labelfrei verwendet werden. Eine geeignete Markierung (Label), insbesondere Isotopenmarkierung kann beispielsweise mittels iTRAQ (Ross et al., Mol Cell Proteomics. 2004;3:1154-1169), ICPL (Kellermann J et al., Methods Mol Biol. 2012;893:143-53), oder Dimethyl-Markierung (Hsu et al., Anal Chem. 2003;75:6843-6852)) erfolgen. Selbstredend können die Aliquots ebenfalls einzeln quantifiziert werden. Die Abtrennung der phosphorylierten Peptide, markiert oder nicht-markiert, erfolgt mittels Titandioxid (Dickhut et al., Methods Mol Biol. 2014;1156:417-30) - alternativ können auch andere Verfahren der Phospopeptidanreicherung wie ERLIC (Loroch et al, Anal Chem, 2015, 3;87(3):1596-604) oder Ti4+-IMAC (De Graaf, Mol Cell Proteomics. 2014;13(9):2426-34) verwendet werden.

Die auf diese Weise abgetrennten phosphorylierten Peptide werden einer quantitativer Massenspektrometrie, vorzugsweise mittels LC-MS/MS zugeführt.

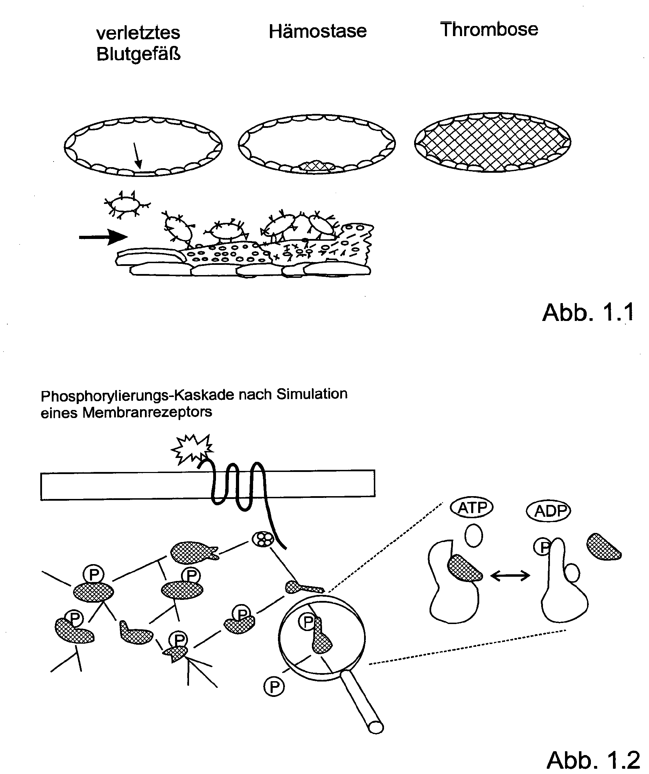

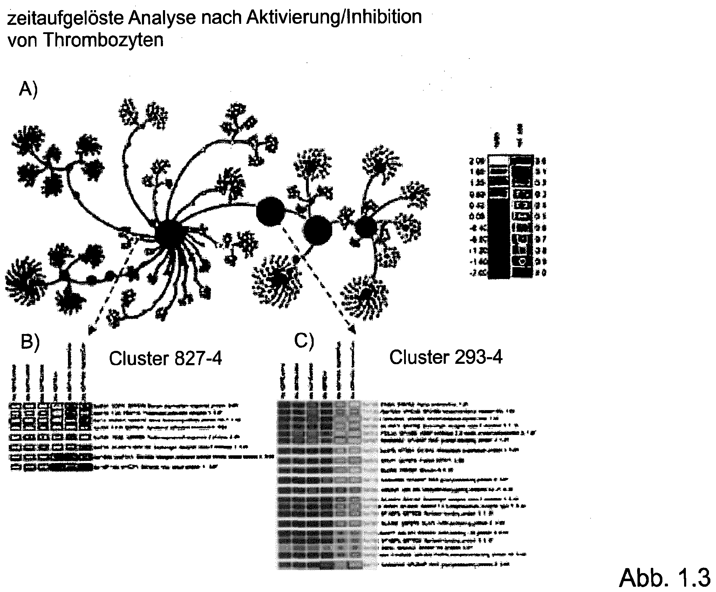

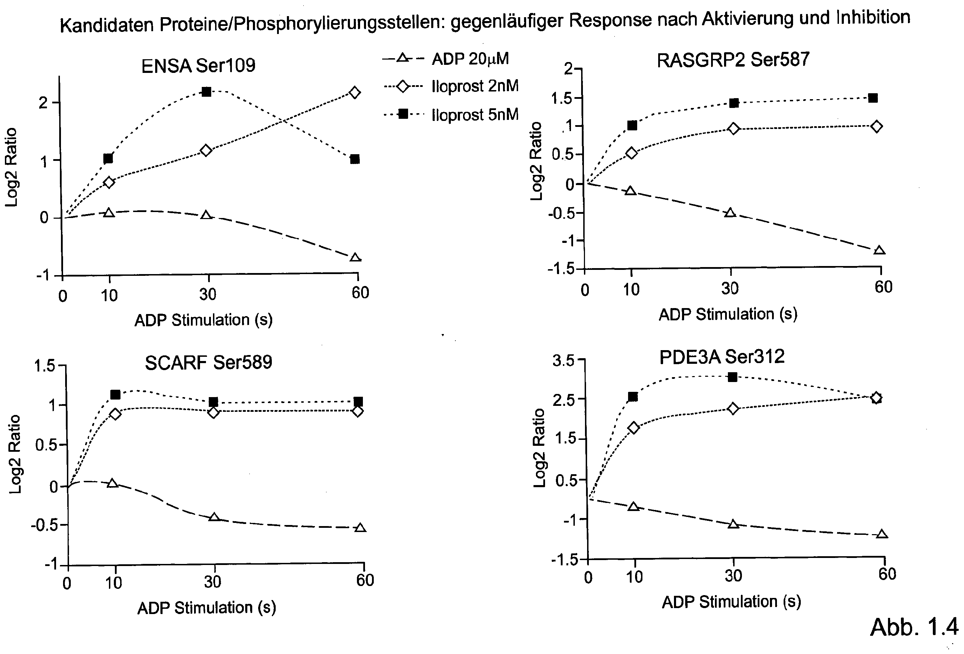

Im Rahmen dieser Erfindung wird unter dem Begriff "quantitativer Massenspektrometrie" eine solche verstanden, wobei "MS/MS" (auch: Tandem-Massenspektrometrie) zur Anwendung kommt. Hierbei werden im Massenspektrometer zunächst MS1 Scans akquiriert, welche die Masse/Ladungsverhältnisse (m/z) der vorhandenen Peptidionen aufzeichnen, und anschließend diese Peptidionen durch Energietransfer z.B. durch Kollision mit Inertgasmolekülen (N2 oder Ar) fragmentiert. Daraufhin zerfallen die Ionen sehr spezifisch zu anderen (leichteren) Ionen, welche im MS/MS Scan ausgelesen werden
Viele Kombinationen der Analysatoren sind verwendbar, solche wie Triplequads (QqQ), QqTOF (Quadropol-Quadrupol-TOF), TOF-TOF als auch TRAP-Orbitrap und weitere Hybdridmassenspektrometer. Neben klassischem MS/MS und MS³ (Ting et al, Nat Meth, 8(11), 937-940 (2011) können MRM (Multiple Reaction Monitoring) und PRM (Parallel Reaction Monitoring) verwendet werden. Weiterhin ist erfindungsgemäß die Kopplung mit einer Flüssigchromatographie (LC) bevorzugt, insbesondere in Form der LC-MS/MS. Insbesondere die LC-MS/MS erlaubt eine zeitaufgelöste quantitative Massenspektrometrie der phosphorylierten Peptide aus Schritt vi.)
Aufgrund der großen Datenmengen (10.000e Spektren, 1000e quantifizierte phosphorylierte Peptide) muss die Auswertung bioinformatisch erfolgen. Hierzu werden die Daten einerseits qualitativ ausgewertet, um die detektierten Peptidsequenzen zu identifizieren (d.h. Aminosäuresequenz bestimmen und Ursprungsprotein zuweisen, Phosphorylierungsstelle innerhalb der Sequenz lokalisieren), und anderseits quantitativ (Auslesen der Signalintensitäten im Massenspektrometer als Maß für die Menge an Peptid). Die Identifizierung erfolgt mit Hilfe von Suchalgorithmen wie Mascot (Perkins DN et al., Electrophoresis. 1999;20:3551), die Lokalisierung der Phosphorylierungsstelle mit Algorithmen wie phosphoRS (Taus et al., J Proteome Res. 2011;10(12):5354-62.) Hierbei werden die Informationen aus den MS und MS/MS Scans mit den Sequenzinformationen aus Proteinsequenzdatenbanken (z.B. erhältlich von www.uniprot.org) abgeglichen. Die Quantifizierung erfolgt abhängig vom letztlich verwendeten Verfahren. Bei Reporter-Ion abhängigen Verfahren (iTRAQ, TMT) werden die aufgrund der Labels generierten Reporter-Signale im MS/MS Spektrum als Maß für die relativen Mengen des Peptids in den unterschiedlichen Proben verwendet (Abbildung 1). Bei nicht-isobaren Labelingverfahren (ICPL, Dimethyl) sowie der labelfreien Quantifizierung erfolgt das Auslesen der quantitativen Information auf Ebene der MS Scans (Abbildung 1). Die Signale werden bioninformatisch in Intensitätswerte umgewandelt (z.B. mit Hilfe der Software Proteome Discoverer von Thermo Scientific) und so können Veränderungen zwischen den Proben quantifiziert werden.

Der erfindungsgemäße Begriff "Störungen der Blutgerinnung" umfasst sämtliche Vorgänge und Prozesse, die insbesondere auf einer nicht-intakten Hämostase beruhen und zu einer verringerten oder erhöhten Blutgerinnung führen. Insbesondere können bei operativen Eingriffen solche Störungen der Blutgerinnung zu lebensbedrohlichen Komplikationen führen. "Störungen der Blutgerinnung" umfasst erfindungsgemäß Krankheiten und Zustände als auch Folgeerkrankungen, wie Thrombose, Schlaganfall, Herzinfarkt, Durchblutungsstörungen sowie kardiovaskuläre Erkrankungen.
Alle genannten Indikationen werden zudem z.B. im Pschyrembel, De Gruyter, 266. Auflage, Berlin 2015 beschrieben.

Der Begriff "Risikostratifizierung" umfasst erfindungsgemäß das Auffinden von Patienten, insbesondere Notfallpatienten und Risikopatienten, mit der schlechteren Prognose, zwecks intensiverer Diagnostik und Therapie/Behandlung von Störungen der Blutgerinnung und verbundenen Erkrankungen als auch Folgeerkrankungen, insbesondere Thrombose, Schlaganfall, Herzinfarkt, Durchblutungsstörungen, kardiovaskuläre Erkrankungen mit dem Ziel einen möglichst günstigen Verlauf dieser Krankheiten zu ermöglichen. Eine erfindungsgemäße Risikostratifizierung erlaubt in Folge ein effektives Behandlungsverfahren, das beispielsweise in eine gezielte Therapiesteuerung münden kann.

Daher betrifft die Erfindung ebenfalls die Identifizierung von Patienten mit erhöhtem Risiko oder/und einer ungünstigen Prognose von Störungen der Blutgerinnung und zwar bei symptomatischen und / oder asymptomatischen Patienten, insbesondere Notfallpatienten.

Besonders vorteilhaft kann insbesondere in Fällen der Notfall- und /oder Intensivmedizin mittels des erfindungsgemäßen Verfahren eine sichere Stratifizierung erfolgen. Das erfindungsgemäße Verfahren ermöglicht daher klinische Entscheidungen, die zu einem schnellen Therapieerfolg und zur Vermeidung von Todesfällen führen. Solche klinischen Entscheidungen umfassen ebenfalls weiterführende Behandlung mittels Arzneimitteln zur Behandlung oder Therapie von Störungen der Blutgerinnung, insbesondere von Thrombozytenaggregationshemmer (TAH), solche wie Abciximab, Acetylsalicylsäure, eine Kombination aus Acetylsalicylsäure und Dipyridamol, Clopidogrel, Eptifibatid, Ilomedin, Prasugrel, Ticagrelor, Ticlopidin, Tirofiban.

Daher betrifft die Erfindung ebenfalls ein Verfahren zur Diagnose und / oder Risikostratifizierung von Patienten von Störungen der Blutgerinnung zur Durchführung von klinischen Entscheidungen, wie weiterführende Behandlung und Therapie mittels Arzneimitteln, vorzugsweise in der zeitlich kritischen Intensivmedizin oder Notfallmedizin, einschließlich der Entscheidung zur Hospitalisierung des Patienten.

In einer weiteren bevorzugten Ausführungsform betrifft das erfindungsgemäße Verfahren daher die Therapiesteuerung von Störungen der Blutgerinnung.

In einer weiteren bevorzugten Ausführungsform des erfindungsgemäßen Verfahrens erfolgt diese zur Diagnose und / oder Risikostratifizierung zur Prognose, zur differentialdiagnostischen Früherkennung und Erkennung, zur Beurteilung des Schweregrades und zur therapiebegleitenden Verlaufsbeurteilung.

In einer weiteren bevorzugten Ausführungsform betrifft die Erfindung ein Verfahren zur in-vitro Diagnostik zur Früh- oder Differentialdiagnose oder Prognose von Störungen der Blutgerinnung an oder von einem zu untersuchenden Patienten.

In einer Ausführungsform des erfindungsgemäßen Verfahrens wird dem zu untersuchenden Patienten Blut entnommen, wahlweise Vollblut oder Serum oder erhältliches Plasma und die Diagnose erfolgt *in vitro*/*ex vivo,* d.h. außerhalb des menschlichen oder tierischen Körpers.

Aufgrund der Bestimmung der phosphorylierten Peptide gemäß dem erfindungsgemäßen Verfahren, wird eine hohe Sensitivität und Spezifität erzielt (siehe Beispiele und Abbildungen) und anhand der vorhandenen Menge entsprechender phosphorylierter Proteine in mindestens einer Patientenprobe gemäß dem erfindungsgemäßen Verfahren kann die Diagnose oder Risikostratifizierung erfolgen.

### Beispiele und Abbildungen:

Nachfolgende Beispiele und Abbildungen dienen zur Erläuterung der Erfindung, ohne diese zu beschränken.

Zunächst werden nach Frischblutentnahme Thrombozyten mittels differentieller Zentrifugation isoliert (Beck et al., Blood 2014, 123(5):e1-e10). Die Probe wird anschließend in Aliquots unterteilt, von denen (i) eines als unbehandelte Kontrolle dient, während (ii-iv) drei mit einem aktivierenden Agonisten wie ADP oder Thrombin für 10 s, 30 s, und 60 s stimuliert werden, bzw. (v-vii) drei inhibiert werden, z.B. durch Zugabe von Iloprost oder Diethylamin-NONOate, ebenso für 10 s, 30 s, 60 s, (viii) eine Probe für 30 s aktiviert und anschließend für 30 s inhibiert wird.

Für die systematische Suche nach geeigneten Markerproteinen sollten verschiedene Konzentrationen, Aktivatoren/Inhbitoren sowie Zeitpunkte gewählt werden. Jede Probe wird direkt im Anschluss an die Stimulation mit einem Lysepuffer (z.B. 2% SDS, Phosphataseinhibitorcocktail) schnell lysiert. Nach der erfolgten Lyse werden die Proteine mittels Ethanol-Präzipitation oder Filter-aided sample preparation (Wisniewski et al., Nat Meth, 6 (2009) 359-362) extrahiert. Es folgt ein proteolytischer Verdau mittels Trypsin (Dickhut et al., J Proteome Res 2014, 6; 13 (6) :2761-70)

Die generierten Peptidproben werden nun entweder mittels stabil-Isotopenmarkierung chemisch gelabelt (z.B. iTRAQ Label von AB Sciex) und anschließend vereint, oder für eine sogenannte markierungsfreie (engl. label free) Analyse einzeln behandelt und analysiert. Um die für die Analyse wichtigen phosphorylierten Peptide vom großen Überschuss an nichtphosphorylierten Peptiden abzutrennen und der Massenspektrometrie zugänglich zu machen, werden phosphorylierte Peptide mittels Titandioxid angereichert (Dickhut et al., Methods Mol Biol. 2014;1156:417-30). Anschließend werden die Phosphopeptidproben entweder einzeln (labelfreie Analyse) oder die vereinte Probe (iTRAQ) mittels nano-LC-MS/MS analysiert (siehe Abbildung 1).

### Abbildung 1: Experimenteller Ablauf nach Isolation und Stimulation (Aktivierung) der Thrombozyten.

Die phosphorylierten Peptide und folglich die Signalantwort der Thrombozyten auf die Aktivierung bzw. Inhibition zeigt sich letztlich bei der Quantifizierung (unten links: Quantifizierung mittels Reporterionen aus den chemischen Labels; unten rechts: "labelfreie" Quantifizierung anhand der Signalintensität des Phosphopeptids in den jeweiligen Proben). (A) Exemplarisches Beispiel für ein potentielles neues Markerprotein. Der Anstieg der Signale ADP 1, ADP2 und ADP3 im Vergleich zur Kontrolle zeigt eine Zunahme der Phosphorylierung nach ADP Stimulation, während die Signale Ilo 1, Ilo 2, Ilo 3 auf eine Abnahme der Phosphorylierung nach Inhibition hindeuten. Die Zunahme durch ADP Stimulation kann letztlich durch Zugabe von Iloprost kompensiert werden (ADP / Ilo). Grundsätzlich ist auch gegensätzlicher Fall möglich, dass ADP zu einer Verringerung und Iloprost zu einem Anstieg führt.

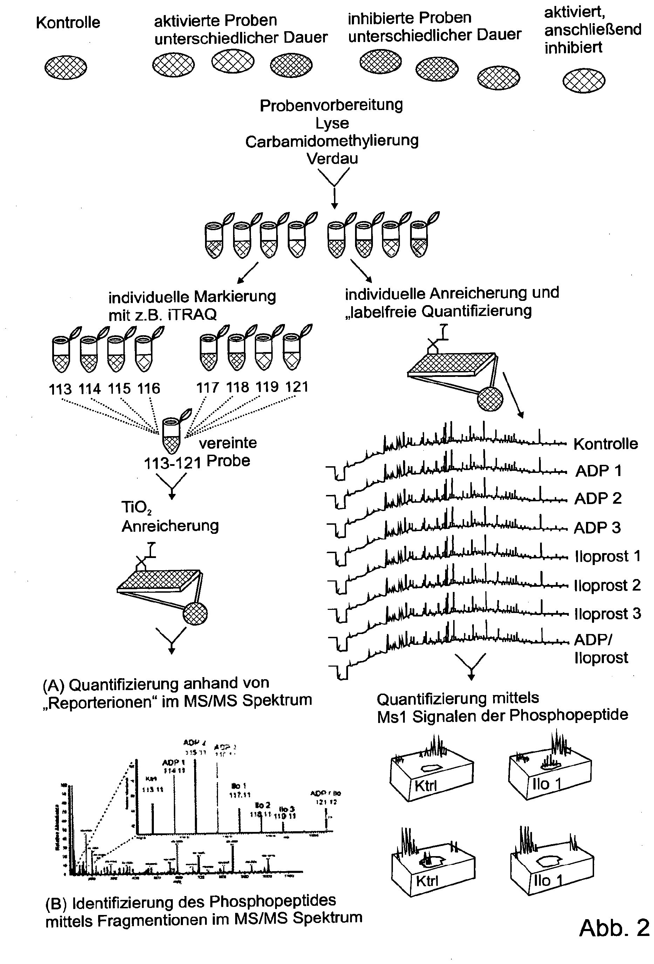

### Abbildung 2: Erläuterung des erfindungsgemäßen Verfahrens.

(i) Thrombozyten nehmen eine Schlüsselrolle im physiologischen Prozess der Hämöstase ein. D.h. wenn ein Blutgefäß verletzt ist, erkennen Thrombozyten dies, werden dadurch "aktiviert", aggregieren und bilden so einen stabilen Thrombus, der die Wunde verschließt, somit den Blutverlust stoppt und auch das Eindringen von Pathogenen und Fremdstoffen verhindert. Dieser Prozess läuft innerhalb weniger Sekunden bis Minuten ab. Im Blutfluss sind u.a. inhibitorisch wirkende Verbindungen vorhanden, die verhindern sollen, dass diese Thrombozytenaktivierung spontan stattfindet. Ist dieses sensible System gestört, kann es z.B. zu Thrombosen kommen, welche Blutgefäße verschließen, damit die Blutversorgung des Gewebes unterbrechen und schließlich zu einem Infarkt führen können.
(ii) Die Aktivierung von Thrombozyten läuft über sogenannte Phosphorylierungskaskaden ab. Aktivierende bzw. inhibierende Komponenten aus dem Blutsystem binden hierbei an Membranrezeptoren, und lösen dadurch ein "Downstream-Signaling" aus. D.h. Enzyme binden gezielt Phosphatreste über kovalente Bindungen an Proteine (Kinasen) bzw. entfernen diese (Phosphatasen). Die Phosphatgruppen führen zu einer Ladungsveränderung an der Oberfläche von Proteinen und somit zu einer Veränderung ihrer dreidimensionalen Struktur. Letzter wiederum definiert, (a) welche Proteine miteinander interagieren, bzw. (b) welche Funktion und (c) Aktivität das Protein letztlich hat. Somit ist die Phosphorylierung ein reversibles und fein-justiertes System zur schnellen Regulation zellulärer Prozesse.
(iii) Mittels quantitativer Massenspektrometrie (MS) können nun Veränderungen der Proteinphosphorylierung hunderter bis tausender Proteine gemessen werden. Hierzu werden Thrombozyten von gesunden Spendern vorzugsweise aus Frischblut isoliert, die ca. 4000-5000 vorhandenen Proteine aufgereinigt und proteolytisch in kleinere Fragmente, sogenannte Peptide, gespalten. Diese Peptide werden einer Anreicherung unterzogen, damit nur solche, die einen oder mehrere Phosphatreste enthalten, der Analyse zugeführt werden. Schließlich wird die quantitative MS dazu genutzt Unterschiede in der Phosphorylierung nach Stimulation von Thrombozyten mit aktivierenden (ADP) bzw. inhibierenden (Iloprost) Verbindungen zu detektieren. Die zeitaufgelösten quantitativen Daten werden bioinformatisch ausgewertet, um Erkenntnisse über das "Netzwerk" (Gesamtheit der Signalwege) zu erhalten. (iv) Die zeitaufgelösten quantitativen Massenspektrometriedaten werden genutzt, um deutlich unterschiedliche (d.h. gegenläufige) Verläufe der Phosphorylierung an bestimmten Proteinen zu detektieren. Selbst wenn die genaue Funktion einer bestimmten Phosphorylierungsstelle an einem Protein nicht bekannt ist, deuten gegensätzliche Verläufe auf eine zentrale Rolle im Gleichgewicht (Homöostase) der Thrombozyten hin. Eine Phosphorylierungsstelle, die bei Aktivierung zunimmt und bei Inhibition abnimmt und vice versa, deutet auf eine zentrale Rolle in beiden Prozessen hin - und stellt damit einen potentiellen Kandidaten (Target) für diagnostische sowie therapeutische Zwecke dar und kann zur Risikostratifizierung von Störungen der Blutgerinnung als auch zur Bestimmung der Aktivierungsgrad und Aktivierbarkeit von Thrombozyten herangezogen werden.

## Patentansprüche

1. Verfahren zur Identifizierung von Markerproteinen zur Diagnose und Risikostratifizierung von Störungen der Blutgerinnung mit den folgenden Schritten:
i.) Bereitstellung von Blut, Vollblut, Blutserum oder Blutplasma enthaltend Thrombozyten,
ii.) Aliquotierung in mindestens zwei Aliquote,
iii.)
A. Zugabe eines Thrombozyten-Aktivators in mindestens ein erstes Aliquot und
B. Zugabe eines Thrombozyten-Inhibitors in mindestens ein zweites Aliquot, und ein oder mehrmalige Wiederholung der jeweiligen Zugabe, und optional
C. Zugabe eines Thrombozyten-Aktivators und Zugabe eines Thrombozyten-Inhibitors in mindestens ein drittes Aliquot oder umgekehrt, und gegebenenfalls ein oder mehrmalige Wiederholung der jeweiligen Zugabe,
iv.) Lyse der Zellen, insbesondere Thrombozyten aus iii.) A. B. und C. im jeweiligen Aliquot,
v.) Extraktion der Proteine und Verdau zu Peptiden mittels Proteasen,
vi.) Anreicherung und Abtrennung der phosphorylierten Peptide,
vii.) Analyse mittels quantitativer Massenspektrometrie und Auswertung,
wobei solche phosphorylierte Peptide ausgewählt werden, die zugleich eine erhöhte oder erniedrigte Phosphorylierung aus iii.) A in der Zeit zeigen und eine gegenläufige erniedrigte oder erhöhte Phosphorylierung aus iii.) B in der Zeit zeigen und optional im Vergleich gegen phosphorylierten Peptiden aus iii.) C eine erniedrigte oder erhöhte Phosphorylierung zeigen.

2. Verfahren zur Bestimmung der Aktivierungsgrad und Aktivierbarkeit von Thrombozyten, **dadurch gekennzeichnet, dass** ein Verfahren nach Anspruch 1 aus Blut von einem Patienten durchgeführt wird.

3. Verfahren nach einem der vorherigen Ansprüche, zur Identifizierung, Diagnose und Risikostratifizierung von Patienten mit erhöhtem Risiko oder/und einer ungünstigen Prognose von Störungen der Blutgerinnung und (Folge)Erkrankungen, insbesondere Thrombose, Schlaganfall, Herzinfarkt, Durchblutungsstörungen sowie kardiovaskuläre Erkrankungen.

4. Verfahren nach einem der vorherigen Ansprüche, wobei der Patient ein symptomatischer und / oder asymptomatischer Patient, insbesondere ein Notfallpatient ist.

5. Verfahren zur Identifizierung, Diagnose und Risikostratifizierung nach einem der vorherigen Ansprüche, zur Therapiesteuerung von Störungen der Blutgerinnung und (Folge-)Erkrankungen, insbesondere Thrombose, Schlaganfall, Herzinfarkt, Durchblutungsstörungen sowie kardiovaskuläre Erkrankungen, insbesondere in der Intensivmedizin oder Notfallmedizin.

6. Verfahren nach einem der vorherigen Ansprüche zur Durchführung von klinischen Entscheidungen, insbesondere weiterführende Behandlungen und Therapien mittels Arzneimitteln, insbesondere in der Intensivmedizin oder Notfallmedizin, einschließlich der Entscheidung zur Hospitalisierung des Patienten.

7. Verfahren nach einem der vorherigen Ansprüche zur Prognose, zur differentialdiagnostischen Früherkennung und Erkennung, zur Beurteilung des Schweregrades und zur therapiebegleitenden Verlaufsbeurteilung.

## Claims

1. A method for identifying marker proteins for the diagnosis and risk stratification of blood coagulation disorders, comprising the following steps:
i.) providing blood, whole blood, blood serum or blood plasma containing thrombocytes,
ii.) aliquoting into at least two aliquots,
iii.)
A. adding a thrombocyte activator to at least a first aliquot and
B. adding a thrombocyte inhibitor to at least a second aliquot, and repeating the addition once or more, and optionally
C. adding a thrombocyte activator and adding a thrombocyte inhibitor to at least a third aliquot, or vice versa, and optionally repeating the respective additions once or more,
iv.) lysing the cells, in particular thrombocytes from iii.) A. B. and C. in the particular aliquot,
v.) extracting the proteins and digesting them to form peptides by means of proteases,
vi.) enriching and separating the phosphorylated peptides,
vii.) performing an analysis by means of quantitative mass spectrometry, and evaluation,
wherein phosphorylated peptides are selected which at the same time demonstrate an increased or reduced phosphorylation from iii.) A over time and which demonstrate, oppositely, reduced or increased phosphorylation from iii. B) over time and which optionally demonstrate a reduced or increased phosphorylation in comparison with phosphorylated peptides from iii.) C.

2. A method for determining the degree of activation and activation ability of thrombocytes, **characterised in that** a method according to claim 1 is performed on a patient's blood.

3. The method according to either one of the preceding claims for identification, diagnosis and risk stratification of patients at increased risk of and/or having an unfavourable prognosis of blood coagulation disorders and (related) diseases, in particular thrombosis, stroke, heart attack, circulatory disorders and cardiovascular diseases.

4. The method according to any one of the preceding claims, wherein the patient is a symptomatic and/or asymptomatic patient, in particular an emergency patient.

5. The method for the identification, diagnosis and risk stratification according to any one of the preceding claims, for therapy management of blood coagulation disorders and (related) diseases, in particular thrombosis, stroke, heart attack, circulatory disorders and cardiovascular diseases, in particular in intensive-care medicine or emergency medicine.

6. The method according to any one of the preceding claims for making clinical decisions, in particular with regard to further treatments and therapies by means of medicinal products, in particular in intensive-care medicine or emergency medicine, including decisions with regard to hospitalisation of the patient.

7. The method according to any one of the preceding claims for prognosis, differential diagnostic early detection and identification, severity assessment and prognostic assessment in conjunction with therapy.

## Revendications

1. Procédé d'identification de marqueurs protéiques pour le diagnostic et la stratification du risque de troubles de la coagulation sanguine avec les étapes suivantes :
i.) mise à disposition de sang, de sang complet, de sérum sanguin ou de plasma sanguin contenant des thrombocytes,
ii.) division en au moins deux fractions aliquotes,
iii.)
A. addition d'un activateur de thrombocytes dans au moins une première fraction aliquote, et
B. addition d'un inhibiteur de thrombocytes dans au moins une deuxième fraction aliquote, et une répétition, ou la répétition à plusieurs reprises, de l'addition respective, et éventuellement
C. addition d'un activateur de thrombocytes et addition d'un inhibiteur de thrombocytes dans au moins une troisième fraction aliquote ou inversement, et éventuellement, une répétition, ou la répétition à plusieurs reprises, de l'addition respective,
iv.) lyse des cellules, notamment des thrombocytes provenant des stades A., B., et C. de l'étape iii) dans la fraction aliquote correspondante,
v.) extraction des protéines et digestion pour former des peptides au moyen de protéases,
vi.) enrichissement et séparation des peptides phosphorylés,
vii.) analyse au moyen de la spectrométrie de masse quantitative et exploitation,
où sont choisis les peptides phosphorylés qui présentent à la fois une phosphorylation augmentée ou diminuée provenant du stade A de l'étape iii.) dans le temps, et une phosphorylation diminuée ou augmentée à contre sens, provenant du stade B de l'étape iii.) dans le temps, et éventuellement, comparativement aux peptides phosphorylés provenant du stade C de l'étape iii.) présentent une phosphorylation diminuée ou augmentée.

2. Procédé de détermination du degré d'activation et du travail d'activation de thrombocytes, **caractérisé en ce qu'**un procédé selon la revendication 1 est exécuté à partir de sang d'un patient.

3. Procédé selon l'une des revendications précédentes pour l'identification, le diagnostic et la stratification du risque de patients ayant un risque élevé et/ou un pronostic défavorable en faveur de troubles de la coagulation sanguine et de maladies pouvant en résulter, notamment de thrombose, d'accident vasculaire cérébral, d'infarctus cardiaque, de troubles circulatoires ainsi que de maladies cardiovasculaires.

4. Procédé selon l'une des revendications précédentes, dans lequel le patient est un patient symptomatique et/ou asymptomatique, notamment un patient en situation d'urgence.

5. Procédé d'identification, de diagnostic et de stratification du risque selon l'une des revendications précédentes, prévu pour la conduite de la thérapie de troubles de la coagulation sanguine et de maladies pouvant en résulter, notamment de thrombose, d'accident vasculaire cérébral, d'infarctus cardiaque, de troubles circulatoires ainsi que de maladies cardiovasculaires, notamment en médecine intensive ou en médecine d'urgence.

6. Procédé selon l'une des revendications précédentes prévu pour l'exécution de décisions d'ordre cliniques, notamment de traitements et de thérapies à poursuivre au moyen de médicaments, en particulier, en médecine intensive ou en médecine d'urgence, y compris la décision d'une hospitalisation du patient.

7. Procédé selon l'une des revendications précédentes prévu pour le pronostic, la reconnaissance précoce et la reconnaissance par diagnostic différentiel, l'évaluation du degré de sévérité et pour l'évaluation du déroulement de la thérapie d'accompagnement.
